(19) **Europäisches Patentamt European Patent Office Office européen des brevets**

(11) **EP 4 676 093 A1**

(12) **EUROPEAN PATENT APPLICATION**

(43) Date of publication:
07.01.2026 Bulletin 2026/02

(21) Application number: 25185612.6

(22) Date of filing: 26.06.2025

(51) International Patent Classification (IPC):
*H04R 17/00* (2006.01)    *H04R 29/00* (2006.01)
*H04R 1/02* (2006.01)

(52) Cooperative Patent Classification (CPC):
**H04R 17/005; H04R 29/001;** A61M 5/14244;
H04R 1/028

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: 01.07.2024   US 202463666590 P
30.05.2025   US 202519224239

(71) Applicant: **Medtronic MiniMed, Inc.
Northridge, CA 91325 (US)**

(72) Inventors:
• **TROCK, Adam S.
Northridge, 91325 (US)**
• **KOUSH, Elizaveta V.
Northridge, 91325 (US)**
• **DIAZ, Daniel L.
Northridge, 91325 (US)**
• **PARARI, Vijaya Kumar
Northridge, 91325 (US)**
• **AYALA, Carlos A.
Northridge, 91325 (US)**
• **SARKESHIK, Shahriar
Northridge, 91325 (US)**

(74) Representative: **Zimmermann & Partner
Patentanwälte mbB
P.O. Box 330 920
80069 München (DE)**

(54) **SYSTEM AND METHOD FOR DETECTION OF PIEZO AUDIO TRANSDUCER FAILURE**

(57) Disclosed herein are techniques related to detecting failures in piezo audio transducers. In some embodiments, the techniques may involve disabling non-essential components and peripherals and forcing microcontroller units into full active mode. The piezo audio transducer is driven at each of a plurality of driving frequencies and, for each driving frequency, the amount of power consumed by the piezo audio transducer is determined. Slope values are determined for each pair or driving frequencies. Based on these slopes, the disclosed techniques generate a piezo health metric for the piezo audio transducer indicating whether the piezo audio transducer passed or failed the test. If the piezo audio transducer fails the test, an appropriate warning or alert can be sent to one or more users and/or other devices.

Figure 5

**Description**

CROSS-REFERENCE TO RELATED APPLICATIONS

This application claims the benefit of priority to U.S. Provisional Application No. 63/666,590, filed July 1, 2024, which is incorporated herein by reference in its entirety.

TECHNICAL FIELD

The present disclosure relates generally to the medical devices and more particularly to detection of piezo transducer failure.

BACKGROUND

Various medical devices are used to deliver therapy to a person, and various other medical devices monitor physiological parameters of a person. For example, a person (e.g., a patient or a user of a diabetes management device) may use insulin therapy to manage type I or type II diabetes. Insulin therapy may include use of insulin infusion systems for delivering or dispensing insulin. An insulin infusion system may include an infusion device which typically includes a small motor and drive train components configured to deliver insulin from a reservoir into the body of a person, e.g., via a percutaneous needle or a cannula placed in the subcutaneous tissue. Insulin infusion systems may facilitate management of diabetes. Medical devices, including insulin infusion systems, may need to communicate, for example, alarms to a user. Often these alarms are audible and/or vibratory in nature and are created by use of a piezo transducer. However, if the piezo transducer is damaged or not otherwise working properly, a user may miss an alarm, which may result in no therapy being delivered, or worse may result in harm to the user. Typical methods of testing for piezo transducer failures rely on user-initiated testing, which some users may not initiate, or periodic system initiated testing that may be inconvenient for the user. Thus, a better testing regime is desirable and would be beneficial.

SUMMARY

Disclosed herein are techniques related to detection of piezo transducer failure in medical devices. The techniques may be practiced in a variety of ways, such as using a processor-implemented method; a system comprising one or more processors and one or more processor-readable media; and/or one or more non-transitory processor-readable media or transitory processor-readable media.

In some embodiments, the techniques for detection of piezo audio transducer failure in medical devices may involve disabling non-essential components and peripherals, such as radio frequency (RF) transmitters, display screens, etc. of the medical device and forcing microcontroller units (MCUs) into full active mode so that, for example, the MCU core is clocked at full system speed and actively executing instructions. The techniques may further involve calculating a background power consumption value corresponding to the amount of power consumed by the medical device when the piezo audio transducer is inactive. In some examples, the disclosed techniques drive the piezo audio transducer at each of a plurality of driving frequencies and, for each driving frequency, determines the amount of power consumed by the piezo audio transducer when being driven at those frequencies. For example, the piezo audio transducer can be driven at 30 kHz, 40 kHz, and 50 kHz and the amount of power consumed by the piezo audio transducer during each of the these driving periods can be determined by measuring the input voltage and current to the piezo audio transducer. For each pair of frequencies (e.g., 30 kHz & 40 kHz, 30 kHz & 50 kHz, and 40 kHz & 50 kHz in this example), the disclosed techniques can calculate a slope value corresponding to the difference between power consumption values for the pair of frequencies divided by the difference between the frequency values. Based on these slopes, the disclosed techniques generate a piezo health metric for the piezo audio transducer indicating whether the piezo audio transducer passed or failed the test. If the piezo audio transducer fails the test, an appropriate warning or alert can be sent to one or more users and/or other devices.

Further disclosed herein are techniques related to detecting failures in piezo audio transducers. In some embodiments, the techniques may involve disabling non-essential components and peripherals and forcing microcontroller units into full active mode. The piezo audio transducer is driven at each of a plurality of driving frequencies and, for each driving frequency, the amount of power consumed by the piezo audio transducer is determined. Slope values are determined for each pair or driving frequencies. Based on these slopes, the disclosed techniques generate a piezo health metric for the piezo audio transducer indicating whether the piezo audio transducer passed or failed the test. If the piezo audio transducer fails the test, an appropriate warning or alert can be sent to one or more users and/or other devices.

BRIEF DESCRIPTION OF THE DRAWINGS

The above and other aspects and features of the disclosure will become more apparent in view of the following detailed description when taken in conjunction with the accompanying drawings wherein like reference numerals identify like elements.

Figure 1 is a diagram of an example therapy delivery system, in accordance with aspects of the present disclosure.

Figure 2 is a diagram of an example insulin delivery device, in accordance with aspects of the present disclosure.

Figure 3A is diagram of an example piezo audio transducer subsystem, in accordance with aspects of the present disclosure.

Figure 3B is diagram of an example piezo audio transducer subsystem, in accordance with aspects of the present disclosure.

Figure 4 illustrates an example method for testing a piezo audio transducer in, accordance with aspects of the present disclosure.

Figure 5 illustrates an example method for calculating a piezo health metric for a piezo audio transducer, in accordance with aspects of the present disclosure.

Figure 6 illustrates an example method for calculating power consumption values, in accordance with aspects of the present disclosure.

Figure 7A illustrates an example method for establishing a test frequency and currency metric of a single frequency piezo audio transducer failure detection system, in accordance with aspects of the present disclosure.

Figure 7B illustrates the current draw of an example piezo audio transducer at a number of driving frequencies.

Figure 8 illustrates an example method for testing a device using a single frequency, in accordance with aspects of the present disclosure.

DETAILED DESCRIPTION

Many devices rely on piezo audio transducers for the generation of audible alerts. Audible alerts serve a critical role during insulin therapy as any interruption to therapy without user notification greatly increases the risk of patient harm due to hyperglycemia. Like any other electromechanical device, a piezo audio transducer can suffer from failures in the field, due to manufacturing defects, accidental or intentional patient misuse or neglect, and so on. Typically, piezo audio transducer failures have not been directly detectable by, for example, an insulin infusion system. Rather, the patient or another user must be relied upon to execute periodic self-tests of the piezo audio transducer and listen for proper operation. Few patients execute these self-tests on a consistent basis, leading to increased risk of piezo audio transducer failures (and an increased risk of patient harm) without detection. Moreover, these self-tests often include an audible test, which can be a nuisance to the patient or user conducting the test and those around them.

Piezo audio transducers typically consume power proportionally to the applied driving voltage and frequency. Thus, as the driving voltage and/or driving frequency increases, so does the amount of power consumed by the piezo audio transducer. However, if the piezo audio transducer is damaged, the piezo audio transducer will appear as an open circuit, short circuit, or resistive shunt, which can drastically change the power consumption of the damaged piezo audio transducer. By determining or estimating the power consumption of the piezo audio transducer at multiple frequencies and performing a mathematical analysis on the resulting measurements, it is possible to determine whether the piezo audio transducer is behaving as an expected capacitive load or whether it is damaged. For example, the piezo audio transducer can be driven at different frequencies (e.g., 30 kHz, 40 kHz, and 50 kHz) and the amount of power consumed can be determined during each of these driving periods. As discussed in further detail below, by comparing the relationship between the amount of power consumed during each of these driving periods, a piezo health metric can be generated for the piezo audio transducer. If the piezo health metric is below a predetermined threshold, a corresponding piezo health variable can be set to "fail" and the patient or another user can be alerted to the potential for damage. For example, the system may alert the user or the user's caregiver via a visible alert on an associated monitoring device, by triggering an alert via a remote system, and so on. In this manner, the piezo audio transducer can be tested periodically without requiring user intervention to initiate or perform the test. Accordingly, the disclosed techniques increase the likelihood of early detection of a piezo audio transducer failure, thereby reducing the risk of harm to a patient who may otherwise not be alerted to problematic blood sugar levels or other concerns.

To avoid audible tests that may be annoying or distracting to those within the vicinity of the device being tested, the driving frequencies used during the periodic tests can be selected from the ultrasonic range (i.e., greater than 20 kHz). Using driving frequencies from the ultrasonic range decreases the potential for humans to be disturbed by the testing, while enabling the capacitive properties of the piezo element to be measured. Moreover, these tests can be limited to short test times to further reduce the disturbance of any pets or other animals capable of hearing in the ultrasonic range.

The disclosed techniques for the detection of piezo audio transducer failure include hardware that facilitates driving a piezo audio transducer at one or more frequencies and determining or estimating the power consumed by the piezo audio transducer or the overall system. For example, many modern battery operated systems include the ability to measure battery voltage and current via pre-existing components of the device, such as a fuel gauging chip, power management integrated circuit (PMIC), and so on. One of ordinary skill in the art will recognize that the power consumed can be determined by multiplying a voltage by current. In some examples, the power consumption determination includes directly measuring the input voltage and current to a piezo driver. In some examples, the power consumption determination can be performed indirectly by measuring the input voltage and current at a battery or other power source. In the event that the measurements are performed indirectly, a background power value (i.e., how much power the system uses when the piezo audio transducer is inactive) can be determined and subtracted from subsequent power calculations.

The disclosed techniques for detection of piezo audio transducer failure determines power consumption during a test sequence that includes driving a piezo audio transducer at one or more driving frequencies (e.g., 40 kHz, 60 kHz, and 70 kHz) and analyzing the results. While one of ordinary skill in the art will recognize that various frequencies may be used to test a piezo audio transducer, and as discussed above, frequencies in the audible range are more likely to disturb humans in the vicinity of the test and the power consumed by each test typically increases as the driving frequency increases. Because these devices are typically battery-operated, energy is at a premium and reducing the number of measurements is generally preferred.

In some examples, the disclosed techniques for detection of piezo audio transducer failure rely on the slope between measurements, where the slope between measurements is defined as the difference between power consumption determined at two different driving frequencies divided by the difference between the driving frequencies. For example, if the power consumption measured at 30 kHz (frequency$_1$) was 250 mW and the power consumption measured at 50 kHz (frequency$_2$) was 348 mW, the slope measurement ($S_{12}$) would be 4.9 (i.e., (348-250)/(50-30) or 98/20)). Because the amount of power consumed by the piezo audio transducer increases as the driving frequency increases, the slope between two power consumption values generated using different frequencies in the piezo audio transducer's operational range should remain generally positive for a healthy piezo audio transducer. The disclosed piezo failure detection system calculates these slope measurements for each pair of driving frequencies and, as discussed in further detail below, uses these slope measurements to diagnose the piezo audio transducer. Because these techniques rely on these slope measurements and do not depend on the absolute amplitude of each measurement, the need for device-specific calibration is eliminated, with the exception of background system power measurement. The background system power measurement can be made at the beginning of each test sequence to remove, for example, any current or power offset from active system devices using the same power source.

In some examples, the disclosed techniques for detection of piezo audio transducer failure use a single frequency to test the health of the piezo audio transducer. Thus, rather than measuring or calculating power consumption at multiple frequencies and analyzing the result, the piezo audio transducer is driven at a predetermined threshold frequency and the amount of input current to the piezo audio transducer is measured. This measured current value is compared to a known current measurement for a healthy device. If the difference between the measured current and the known current measurement for the healthy device is within an acceptable range (e.g., +/-5%, +/- 10%, +/- 20%) then the piezo audio transducer is considered healthy.

One challenge with using battery power consumption as a proxy for piezo audio transducer current is the need to filter out or eliminate the additional power consumption of the other system components, such as processing devices (i.e., MCUs, memories, FPGAs), radio communications (i.e., Bluetooth®, Wi-Fi, LTE), and any other components that consume nonnegligible battery power (i.e., displays, motors, sensors, LEDs). While system components can be temporarily disabled or placed into constant power or current modes to simplify filtering, radio communications may present an additional challenge if the system requirements prevent total disabling of the radio. Radio frequency (RF) protocols such as Bluetooth® and Wi-Fi consist of frequent transmit/receive bursts that are highly variable in both power level and period. Device control systems, such as host controllers, are often unaware of the exact timing of this RF activity, as the intervals are typically managed at the communication hardware level. Furthermore, even if the radio frequency activity period (RFAP) is known (e.g., an RFAP determined at the time of design), the specific timing of the corresponding RF activity may not. As a result, it may not be possible to schedule the piezo audio transducer sampling to guarantee avoidance of all RF activity.

In some examples, the disclosed techniques for detection of piezo audio transducer failure address this potential RF interference issue in two ways: (1) repeating the measurement of one or more driving frequencies used in the test sequence and (2) ensuring that the least common multiple (LCM) of the RFAP and piezo sampling period (PSP) (i.e., the period between samples at different frequencies) is at least *n+1* times greater than the PSP, wherein *n* is the number of frequencies included in the test sequence. For example, if the test includes determining power consumption at 30 kHz, 40 kHz, and 50 kHz (i.e., three frequencies), then *n* would be three and the LCM of the RFAP and piezo sampling period is at least four (*n+1*) times greater than the PSP. In this manner, the frequency that is most likely to be susceptible to RF interference (i.e., the lowest frequency) will be tested twice and the sample with the largest value from among those two samples (i.e., the sample that is more likely to have been measured during an RF burst or other activity) is discarded. However, because the PSP is calculated to ensure that the LCM of the RFAP and PSP is at least *n+1* times greater than the PSP, at least one of the samples at the lowest driving frequency will not overlap with RF activity (where the RFAP is known). In this manner, the disclosed techniques provide robust piezo transducer failure detection while ensuring a high level of immunity from RF activities that coincide with piezo measurements during the testing of piezo audio transducers.

The present disclosure is described primarily with respect to insulin delivery systems. Aspects and embodiments of the present disclosure can be practiced with one or more types of insulin (e.g., fast-acting insulin, intermediate-acting insulin, and/or slow-acting insulin). Although the present disclosure is described primarily with respect to insulin delivery systems, the scope of the present disclosure is not limited to insulin delivery systems. Rather, the present disclosure applies to and can be implemented for other systems as well. For example, in some embodiments, the techniques in the present disclosure may be practiced with other medical devices, for example pain medicine infusion pumps, medical monitoring systems, therapy delivery systems, etc. Further, the techniques of the present disclosure may be practiced in relation to piezo audio transducers used in other, non-medical related fields, such as smoke detectors, carbon monoxide detectors, motion detectors, gas leak monitors, air quality monitors (e.g., indoor or outdoor), leak detectors (e.g., water or gas), and so on.

Discussions utilizing terms such as, for example, "processing," "computing," "calculating," "determining," "establishing," "analyzing," "checking," or the like, may refer to operation(s) and/or process(es) of a computer, a computing platform, a computing system, or other electronic computing device, that manipulate and/or transform data represented as physical (e.g., electronic) quantities within the computer's registers and/or memories into other data similarly represented as physical quantities within the computer's registers and/or memories or other non-transitory information storage media that may store instructions to perform operations and/or processes.

Unless explicitly stated, the methods described herein are not constrained to a particular order or sequence. Additionally, some of the described methods or elements thereof can occur or be performed simultaneously or concurrently.

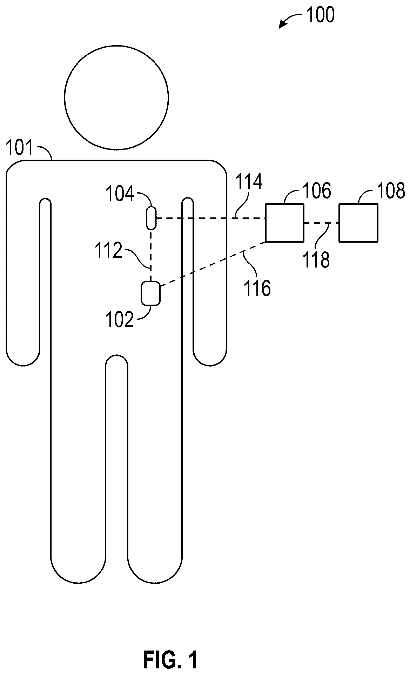

Figure 1 depicts an example therapy delivery system 100 for a person 101. System 100 may be an insulin delivery system. The depicted therapy delivery system 100 includes a delivery device 102, a monitoring device 104, a computing device 106, and an optional remote or cloud computing system 108. The delivery device 102, the monitoring device 104, and the computing device 106 may be embodied in various ways, including being disposed in one or more device housings. For example, in some embodiments, all of the devices 102-106 may be disposed in a single device housing. In some embodiments, each of the devices 102-106 may be disposed in a separate device housing. In some embodiments, two or more of the devices 102-106 may be disposed in the same device housing, and/or a single device 102, 104, or 106 may have two or more parts that are disposed in two or more housings. Such embodiments, and combinations thereof, are contemplated to be within the scope of the present disclosure.

Figure 1 also depicts communications links 112, 114, 116 and 118. The communications links 112, 114, 116, 118 may each be a wired connection and/or a wireless connection. In the case where two devices are located in the same device housing, the communication link may include, for example, wires, cables, and/or communication buses on a printed circuit board, among other things. In the case where two devices are separated from each other in different device housings, the communication links may be wired and/or wireless connections. Wired connections may include, without limitation, an Ethernet connection, a USB connection, and/or another type of physical connection. Wireless connections may include, without limitation, a cellular connection, a Wi-Fi connection, a Bluetooth® connection, a mesh network connection, and/or another type of connection using a wireless communication protocol. Some embodiments of the communication links 112, 114, 116, 118 may use direct connections, such as Bluetooth® connections, and/or may use connections that route through one or more networks or network devices (not shown), such as an Ethernet network, a Wi-Fi network, a cellular network, a satellite network, an intranet, an extranet, the Internet, and/or the Internet backbone, among other types of networks. Various combinations of wired and/or wireless connections may be used for the communication links 112, 114, 116, 118.

Aspects of exemplary insulin delivery systems 100 are described below. An exemplary delivery device 102 is configured to deliver a therapeutic substance (e.g., insulin) to a person 101. The delivery device 102 may be secured to the person 101 (e.g., to the body or clothing of the person 101) or may be implanted on or in the body of the person 101. In some embodiments, the delivery device 102 may include a reservoir, an actuator, a delivery mechanism, and a cannula (not shown). The reservoir may be configured to store an amount of the therapeutic substance. In some embodiments, the reservoir may be refillable or replaceable. The actuator may be configured to drive the delivery mechanism. In some examples, the actuator may include a motor, such as an electric motor. The delivery mechanism may be configured to move the therapeutic substance from the reservoir through the cannula. In some examples, the delivery mechanism may include a pump and/or a plunger. The cannula may facilitate a fluidic connection between the reservoir and the body of the person 101. The cannula and/or a needle may facilitate delivery of the therapeutic substance to a tissue layer, vein, or body cavity of the person 101. During operation, the actuator, in response to a signal (e.g., a command signal), may drive the delivery mechanism, thereby causing the therapeutic substance to move from the reservoir, through the cannula, and into the body of the person 101.

The components of the delivery device 102 described above are merely provided as examples. The delivery device 102 may include other components, such as, without limitation, a power supply, a communication transceiver, computing resources, and/or user interfaces, among other things. Persons skilled in the art will recognize various implementations of the delivery device 102 and the components of such implementations. All such implementations and components are contemplated to be within the scope of the present disclosure.

With continuing reference to Figure 1, the exemplary monitoring device 104 is configured to detect a physiological condition (e.g., a glucose concentration level) of the person 101 and may also be configured to detect other things. The monitoring device 104 may be secured to the body of the person 101 (e.g., to the skin of person 101 via an adhesive) and/or may be at least partially implanted into the body of the person 101. Depending on the particular location or configuration, the monitoring device 104 may be in contact with biological matter (e.g., interstitial fluid and/or blood) of the person 101.

The monitoring device 104 may include one or more sensors (not shown), such as, without limitation, electrochemical sensors, electrical sensors, and/or optical sensors. As persons skilled in the art will understand, an electrochemical sensor may be configured to respond to the interaction or binding of a biological marker to a substrate by generating an electrical signal based on a potential, conductance, and/or impedance of the substrate. The substrate may include a material selected to interact with a particular biomarker, such as glucose. The potential, conductance, and/or impedance may be proportional to a concentration of the particular biomarker.

The monitoring device 104 may include components and/or circuitry configured to pre-process sensor signals. Pre-processing may include, without limitation, amplification, filtering, attenuation, scaling, isolation, normalization, transformation, sampling, and/or analog-to-digital conversion, among other things. Persons skilled in the art will recognize various implementations for such pre-processing, including, without limitation, implementations using processors, controllers, ASICS, integrated circuits, hardware, firmware, programmable logic devices, and/or machine-executable instructions, among others. The types of pre-processing and their implementations are merely provided as examples. Other types of pre-processing and implementations are contemplated to be within the scope of the present disclosure. In some embodiments, the monitoring device 104 may not perform pre-processing.

The computing device 106 provides processing capabilities and may be implemented in various ways. In some embodiments, the computing device 106 may be a consumer device, such as a smartphone, a computerized wearable device (e.g., a smartwatch), a tablet computer, a laptop computer, or a desktop computer, among others, or may be a special purpose device (e.g., a portable control device) provided by, for example, the manufacturer of the delivery device 102. In some embodiments, the computing device 106 may be "processing circuitry" (defined below) that is integrated with another device, such as the delivery device 102. In some embodiments, the computing device 106 may be secured to the person 101 (e.g., to the body or clothing of person 101), may be at least partially implanted into the body of person 101, and/or may be held by the person 101.

For each of the embodiments of the computing device 106, the computing device 106 may include various types of logic circuitry, including, but not limited to, microprocessors, controllers, digital signal processors (DSPs), application specific integrated circuits (ASICs), field-programmable gate arrays (FPGAs), central processing units (CPU), graphics processing units (GPU), programmable logic devices, memory (e.g., random access memory, volatile memory, nonvolatile memory, etc.), or other discrete or integrated logic circuitry, as well as combinations of such components. The term "processing circuitry" may generally refer to any of the foregoing logic circuitry, alone or in combination with other logic circuitry, or any other circuitry for performing computations.

Aspects of the delivery device 102, the monitoring device 104, and the computing device 106 have been described above. One or more of the devices 102, 104, and 106 may include a user interface (not shown) that presents information to the person 101 and/or receives information from the person 101. The user interface may include a graphical user interface (GUI), a display device, a keyboard, a touchscreen, a speaker, a microphone, a vibration motor, buttons, switches, and/or other types of user interfaces. Persons skilled in the art will recognize various types of user interfaces that may be used, and all such user interfaces are contemplated to be within the scope of the present disclosure. For example, where the computing device 106 is a consumer device such as a smart phone, tablet computer, laptop computer, or the like, the user interfaces would include a display device, a physical and/or virtual keyboard, and/or audio speakers provided by such consumer devices, among other things. In some embodiments, a user interface may notify the person 101 of sensed data (e.g., glucose level) and/or insulin delivery data (e.g., rates of historic, current, or future insulin delivery) and may provide alerts to the person 101 such as visible alerts via a display device, audible alerts via a transducer (e.g., piezoelectric audio transducer), and so on. In some embodiments, a user interface may receive inputs from the person 101, which may include, for example, a requested change in insulin delivery and/or a meal indication, among other things. The descriptions and embodiments above regarding user interfaces are merely provided as examples, and other types and other uses of user interfaces are contemplated to be within the scope of the present disclosure.

The following describes communications between the devices 102, 104 and 106 and cooperation between the devices 102, 104 and 106 with respect to insulin delivery. As depicted in Figure 1, and as mentioned above, the devices 102, 104 and 106 may communicate with each other via communication links 112, 114 and 116. In some embodiments, the computing device 106 may control operation of the delivery device 102 and/or the monitoring device 104. For example, the computing device 106 may generate one or more signals (e.g., a command signal) that cause the delivery device 102 to deliver insulin to the person 101, e.g., as a basal dosage and/or a bolus dosage. In some embodiments, the computing device 106 may receive data associated with insulin delivery (e.g., insulin delivery data) from the delivery device 102 and/or receive sensed data (e.g., glucose levels) from the monitoring device 104 and may perform computations based on the insulin delivery data, the sensed data, and/or other data to control the delivery device 102. Insulin delivery data may include, but is not limited to, a type of insulin being delivered, historical insulin delivery rates and/or amounts, current insulin delivery rate and/or amount, and/or user input affecting insulin delivery. As persons skilled in the art will understand, in a closed-loop operating mode, computing device 106 may communicate dosage commands to the delivery device 102 based on a difference between a current glucose level in the body of the person 101 (e.g., received from the monitoring device 104) and a target glucose level (e.g., determined by the computing device 106). The dosage commands may indicate an amount of insulin to be delivered and/or a rate of insulin delivery and may regulate the current glucose level toward the target glucose level.

With continuing reference to Figure 1, the remote or cloud computing system 108 may be a proprietary remote/cloud computing system or a commercial cloud computing system including one or more server computing devices. The remote/cloud computing system 108 may provide additional computing resources on-demand as needed when the computing resources of a client computing device (e.g., the computing device 106) are not sufficient. The computing device 106 and the remote/cloud computing system 108 may communicate with each other through a communication link 118, which may traverse one or more communication networks (not shown). The communication networks may include, without limitation, an Ethernet network, Wi-Fi network, a cellular network, a satellite network, an intranet, an extranet, the Internet, and/or the Internet backbone, among other types of networks. Persons skilled in the art will recognize implementations for the remote/cloud computing system 108 and how to interface with such systems through various types of networks. For example, the remote/cloud computing system 108 may include an array of processing circuitry (defined above) and may execute machine-readable instructions. Such implementations, interfaces, and networks are contemplated to be within the scope of the present disclosure.

The computing devices can access computer-readable media that includes computer-readable storage media and data transmission media. The computer-readable storage media are tangible storage means that do not include transitory, propagating signals. Examples of computer-readable storage media include memory such as primary memory, cache memory, and secondary memory (e.g., CD, DVD, Blu-Ray) and include other storage means. Moreover, data may be stored in any of a number of data structures and data stores, such as a databases, files, lists, emails, distributed data stores, storage clouds, etc. The computer-readable storage media can have recorded upon or can be encoded with computer-executable instructions or logic that implements the disclosed techniques, such as a component comprising computer-executable instructions stored in one or more memories for execution by one or more processors. In addition, the stored information can be encrypted. The data transmission media are used for transmitting data via transitory, propagating signals or carrier waves (e.g., electromagnetism) via a wired or wireless connection. In addition, the transmitted information can be encrypted.

An example therapy delivery system has been described above as an example. For convenience, the description below may primarily refer to an insulin delivery system as an example of the therapy delivery system. However, it is intended that any aspect, embodiment, or description relating to an insulin delivery system shall be applicable to a therapy delivery system which delivers a therapy other than insulin. Further, the present invention may be applied to devices having a piezo transducer other than therapy delivery systems.

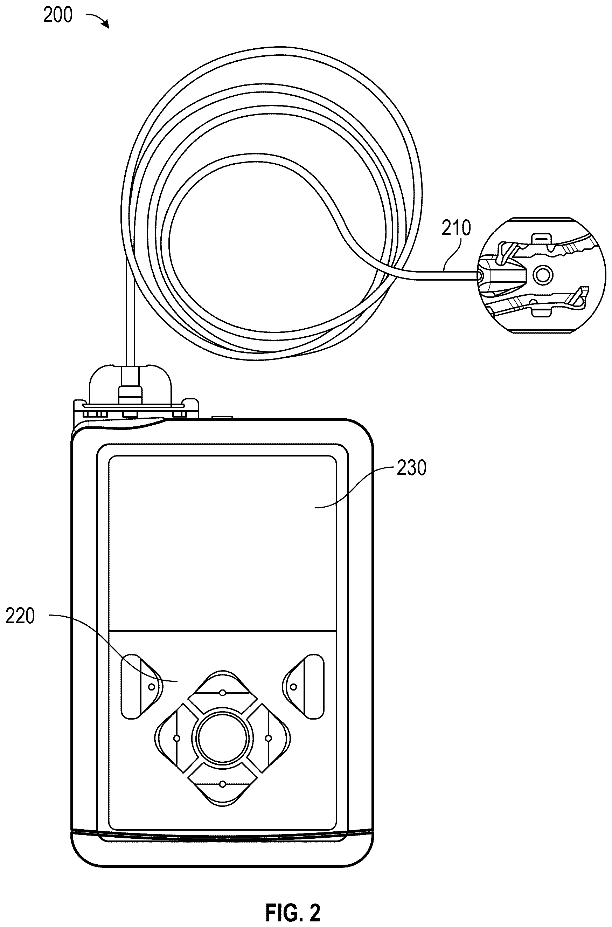

In some embodiments, therapy may be effected based on communicating a therapy determination toward a therapy delivery device. A non-limiting example of such a device is described below in connection with Figure 2. The insulin delivery device 200 can provide fast-acting insulin through a small tube 210 configured for fluidic connection with a cannula inserted subcutaneously. The device 200 can deliver two types of dosages-a basal dosage, which can be delivered periodically (e.g., every five minutes) in tiny amounts throughout the day and night, and a bolus dosage to cover an increase in blood glucose from meals and/or to correct high blood glucose levels. The depicted insulin delivery device 200 includes a user interface having button elements 220 that can be manipulated to administer a bolus of insulin, to change therapy settings, to change user preferences, to select display features, and the like. The insulin delivery device 200 also includes a display device 230 that can be used to present various types of information or data to the user. In accordance with aspects of the present disclosure, a user of the insulin delivery device 200 may use the button elements 220 to input certain event data (e.g., event type, event start time, event details, etc.), and the user inputs can be confirmed using the display device 230. The depicted insulin delivery device 200 of Figure 2 is merely provided by way of example, and other types of insulin delivery devices and other techniques different from those described above are contemplated to be within the scope of the present disclosure.

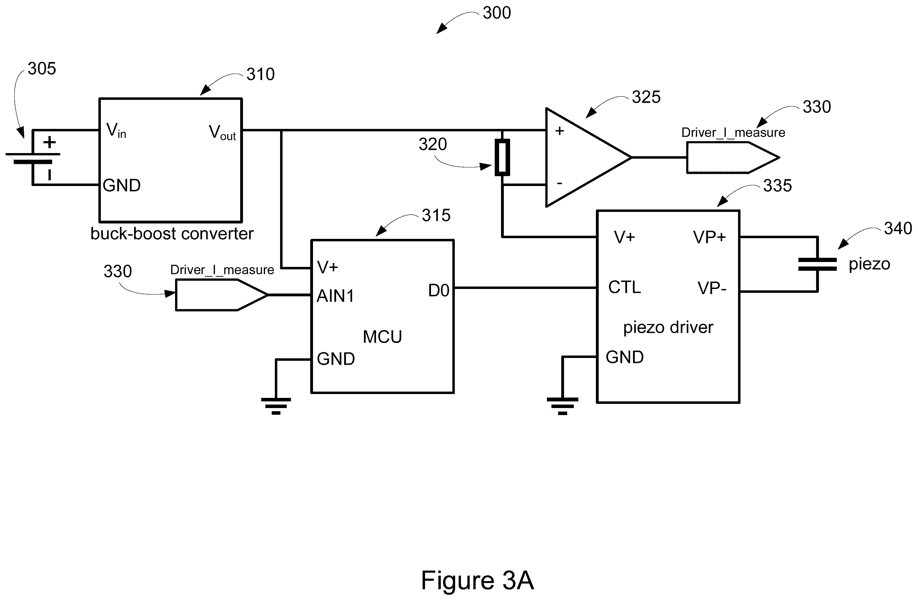

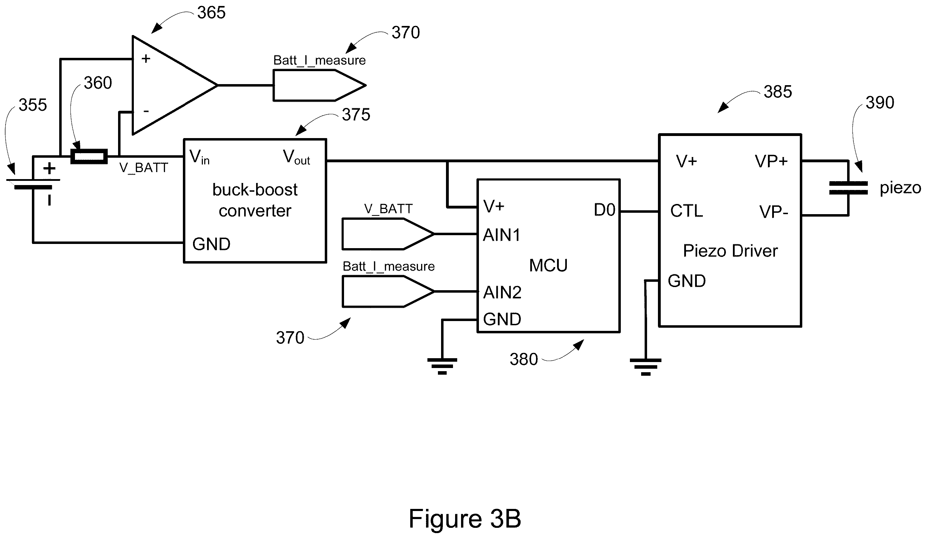

Figures 3A and 3B are diagrams of example piezo audio transducer subsystems, in accordance with aspects of the present disclosure. As discussed above, many modern battery operated systems include the ability to measure battery voltage and current via pre-existing components (e.g., a fuel gauging chip, PMIC, and so on). However, if a system does not include the ability to measure battery voltage and/or current, hardware can be added to the system so that power and/or current measurements can be taken. Figure 3A represents an example of hardware that can be included to facilitate the direct measurement of piezo current (i.e., input current to the piezo driver). In this example, piezo audio transducer subsystem 300 comprises battery 305, buck-boost converter 310, microcontroller unit 315, resistor 320, operational amplifier 325, piezo driver 335, and piezo transducer 340. The input current to the piezo driver is determined by operational amplifier 325 across resistor 320 and output as "Driver_I_measure" to microcontroller unit 315. Figure 3B represents an example of hardware that can be included to facilitate the indirect measurement of piezo audio transducer power consumption by measuring the voltage and total system current at the battery. In this example, piezo audio transducer subsystem 350 comprises battery 355, resistor 360, operational amplifier 365, buck-boost converter 375, microcontroller unit 380, piezo driver 385, and piezo transducer 390. In this example, the battery current is determined by operational amplifier 365 across resistor 360 and output as "Batt_I_measure" to microcontroller unit 380 along with V_BATT, the voltage of battery 355. One of ordinary skill in the art will recognize that the piezo audio transducer subsystems illustrated in Figures 3A and 3B are integrated into a larger system that includes additional components (not shown) that operate in conjunction with the piezo audio transducer subsystem, such as a therapy delivery system (e.g., an insulin infusion system), a medical monitoring system, or any other medical device. Moreover, one of ordinary skill in the art will recognize that these examples are illustrative and that other techniques and/or hardware for measuring current, voltage, and/or power can be used.

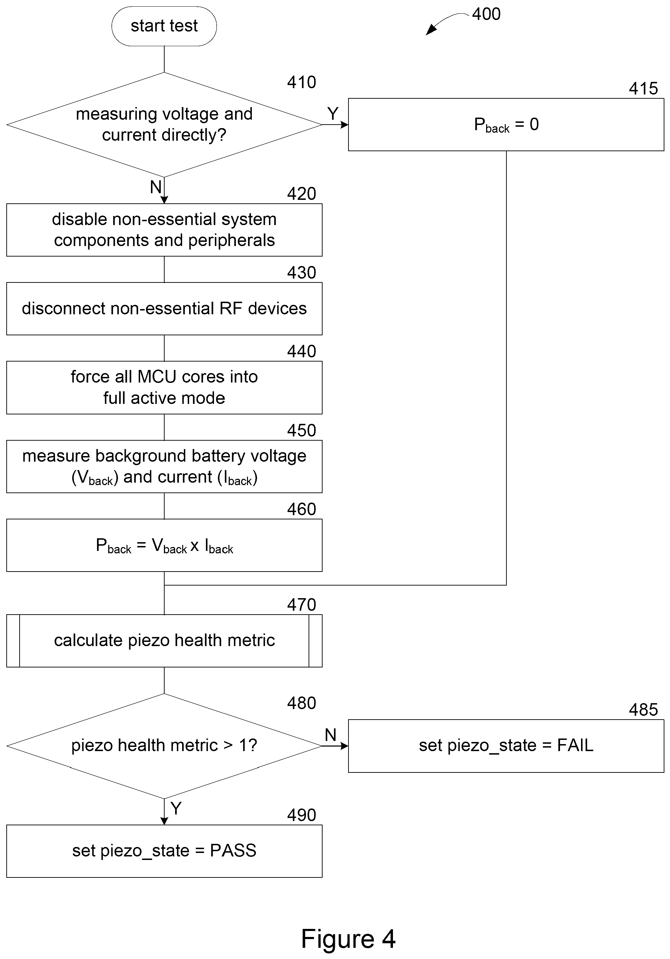

Figures 4-8 illustrate example methods in accordance with the present technology. The methods 400, 500, 600, 700, and 800 can be implemented by any of the devices described herein, or any other suitable devices now known or later developed, that utilize a piezo transducer. Various embodiments of the methods 400, 500, 600, 700, and 800 include one or more operations, functions, or actions illustrated by blocks. Although the blocks are illustrated in sequential order, these blocks may also be performed in parallel, and/or in a different order than the order disclosed and described herein. Also, the various blocks may be combined into fewer blocks, divided into additional blocks, and/or removed based upon a desired implementation.

In addition, for the methods 400, 500, 600, 700, and 800 and for other processes and methods disclosed herein, the flowcharts show functionality and operation of possible implementations of some embodiments. In this regard, each block may represent a component, a module, a segment, or a portion of program code, which includes one or more instructions executable by one or more processors for implementing specific logical functions or steps in the process. The program code may be stored on any type of computer readable medium, for example, such as a storage device including a disk or hard drive. The computer readable storage media may include non-transitory computer readable media, for example, such as tangible, non-transitory computer-readable media that store data for short periods of time like register memory, processor cache, and Random-Access Memory (RAM). The computer readable storage media may also include non-transitory media, such as secondary or persistent long-term storage, like read only memory (ROM), optical or magnetic disks, compact disc read only memory (CD-ROM), for example. The computer readable storage media may also be any other volatile or non-volatile storage systems. The computer readable medium may be considered a computer readable storage medium, for example, or a tangible storage device. In addition, for the methods and for other processes and methods disclosed herein, each block in Figures 4-8 may represent circuitry that is wired to perform the specific logical functions in the process.

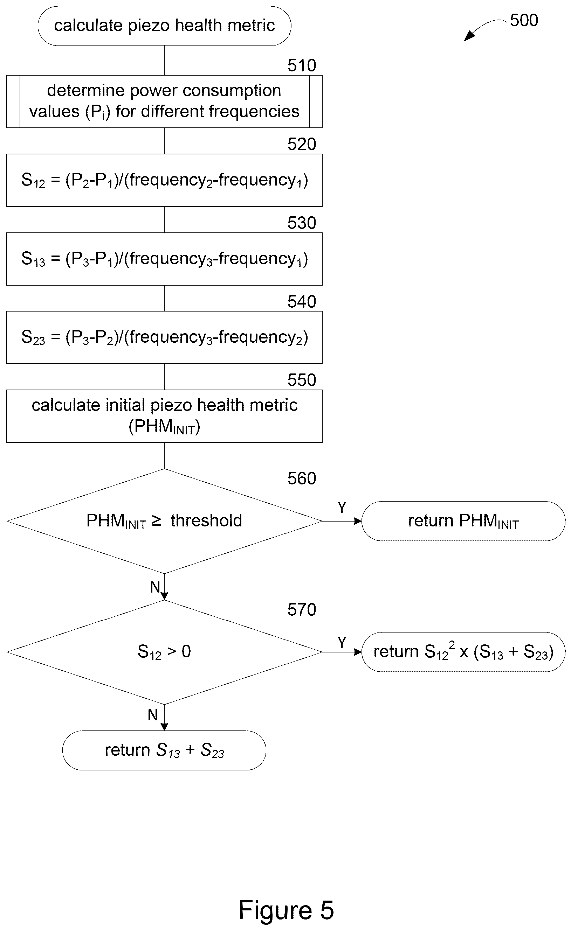

Figure 4 illustrates an example method 400 for testing a piezo audio transducer of a device, in accordance with aspects of the present disclosure. The method 400 begins at decision block 410, which involves determining whether the test sequence will include measuring piezo voltage and current directly or indirectly. As discussed above, measuring piezo voltage and current indirectly (i.e., via the device's battery) can introduce noise from other components using the device's battery. In decision block 410, if the test sequence will involve direct measurements, then the method continues at block 415, otherwise the method continues at block 420. In blocks 420-460 the method attempts to reduce the amount of background noise generated by other components. In block 420, the method disables certain non-essential system components and peripherals, such as display devices, motors, lights, etc. In block 430, the method disconnects non-essential RF devices by, for example, sending a request to a control system to shut down RF devices when available. In block 440, the method forces all microcontroller unit cores into full active mode to increase the rate at which the test sequence can be performed, thereby limiting the amount of time the non-essential elements are disabled and/or disconnected. Although it may not be feasible to completely eliminate all background noise, any decrease is likely to improve test results. In block 450, the method measures the background battery voltage ($V_{back}$) and background battery current ($I_{back}$). As discussed above, these measurements can be performed via any number of measurement techniques, such as via a fuel gauging chip, PMIC, or other hardware. In block 460, the method sets a variable "$P_{back}$," that represents the background power consumption of the device (i.e., the amount of power consumed from the battery while the devices disconnected or disabled in blocks 420 and 430 are not in use) by multiplying $V_{back}$ by $I_{back}$. In block 415, the method sets $P_{back}$ to 0 because the test sequence will include measuring piezo voltage and current directly and, therefore, the power consumption of the piezo will be less susceptible to noise generated by the consumption of power by other components. In block 470, the method invokes a sub-routine (discussed below with reference to Figure 5) to calculate a piezo health metric (PHM) for the piezo audio transducer. In decision block 480, if the calculated piezo health metric is greater than 1, then the method continues at block 490, otherwise method continues at block 485. In block 485, the method sets a piezo_state variable to FAIL. In some cases, the method may also send an alert or indication of the error to one or more parties or devices to indicate the audio transducer failed the test. For example, the method may send an alert to a paired device, such as a display screen, phone, tablet, etc. As another example, the method may send a message to a phone number or email address of the patient, the patient's caregiver, or another party. As another example, the method may cause a light emitting diode (LED) or other visible indicator to flash or blink to alert a user or other individual. In block 490, the method sets the piezo_state variable to PASS. After the test is performed, the method may terminate or loop back to decision block 410 to perform the test periodically (e.g., every 4 hours, 12 hours, daily, weekly, etc.).

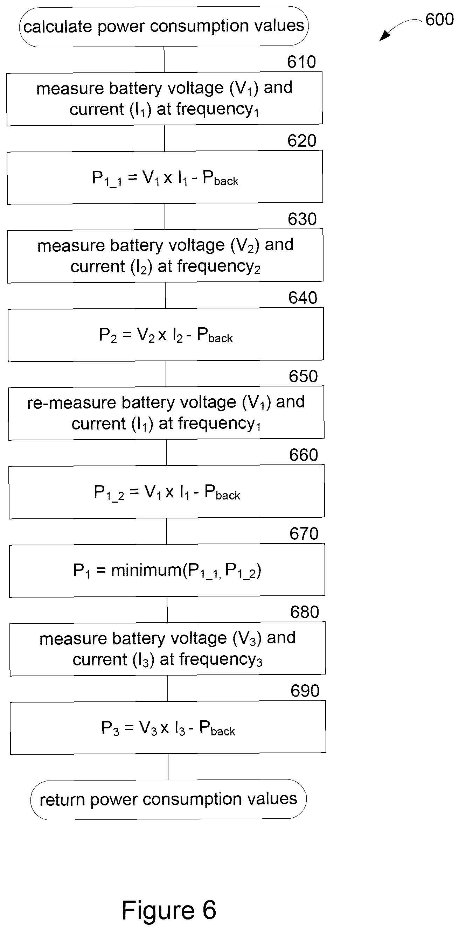

Figure 5 illustrates an example method 500 for calculating a piezo health metric for a piezo audio transducer, in accordance with aspects of the present disclosure. The method 500 begins at block 510 by invoking a sub-routine (discussed below with reference to Figure 6) to determine, for each of a number of driving frequencies, the amount of power consumed by the piezo audio transducer when being driven at the corresponding frequency. In this example, the test sequence involves testing three different driving frequencies: $frequency_1$, $frequency_2$, and $frequency_3$. For ease of reading, the power consumption value generated when driving the piezo audio transducer at $frequency_1$ is labeled "$P_1$," the power consumption value generated when driving the piezo audio transducer at $frequency_2$ is labeled "$P_2$," and so on. Furthermore, the slopes between the power consumption values are designated with an "S" and a subscript corresponding to the two frequencies used to generate the two power consumption values represented by the close. For example, the slope between power consumption values generated via $frequency_1$ and $frequency_2$ is labeled "$S_{12}$," the slope between the power consumption values generated for $frequency_2$ and $frequency_3$ is labeled "$S_{23}$," and so on. In block 520, the method calculates $S_{12}$ as $(P_2-P_1)/(frequency_2-frequency_1)$. In block 530, the method calculates $S_{13}$ as $(P_3-P_1)/(frequency_3-frequency_1)$. In block 540, the method calculates $S_{23}$ as $(P_3-P_2)/(frequency_3-frequency_2)$. In this manner, the method generates slope values for each combination of frequencies used during the test. As discussed above, one of ordinary skill in the art will recognize that additional driving frequencies may be used, and corresponding slopes calculated according to these equations (i.e., $S_{xy} = (P_y-P_x)/(frequency_y-frequency_x)$). In block 550, the method generates an initial piezo health metric ($PHM_{INIT}$) for the piezo audio transducer based on the generated slope values. In one example, the method calculates $PHM_{INIT}$ as

$$PHM_{INIT} = MAXIMUM(S_{12}, S_{13}, S_{23}) * MINIMUM\_POSITIVE(S_{12}, S_{13}, S_{23})^2,$$

where "MAXIMUM" represents a function that returns the maximum or largest value from a set of values and "MINIMUM_POSITIVE" represents a function that returns the minimum or smallest value that is greater than 0 from a set of values. In this manner, the method generates an initial piezo health metric that represents the product of the largest value from among $S_{12}$, $S_{13}$, and $S_{23}$ and the square of the smallest positive value from among $S_{12}$, $S_{13}$, and $S_{23}$. In decision block 560, if the initial piezo health metric is greater than or equal to a predetermined threshold value, then the method returns (to decision block 480) the initial piezo health metric as the piezo health metric (PHM) for the piezo audio transducer, otherwise the method continues at decision block 570. In some examples, the predetermined threshold is determined by testing a number (e.g., 10, 50, 100) of known (or presumed) healthy devices and calculating an average (e.g., mean, median, mode) $PHM_{INIT}$ for those devices and a corresponding standard deviation. In this manner, the predetermined threshold can be defined to include device's with a $PHM_{INIT}$ that is within a fixed number (e.g., 1, 1.5, 2, 2.5) of standard deviations from the calculated average. In decision block 570, if $S_{12}$ is greater than 0, then the method returns (to decision block 480) the product of $S_{12}$ squared and the sum of $S_{13}$ and $S_{23}$ as the PHM for the piezo audio transducer, otherwise the method returns (to decision block 480) the sum of $S_{13}$ and $S_{23}$ as the PHM for the piezo audio transducer. In this manner, the PHM for a piezo audio transducer is generated based on the slopes between different power consumption values and corresponding driving frequencies.

Figure 6 illustrates an example method 600 for calculating power consumption values, in accordance with aspects of the present disclosure. Method 600 is invoked to drive the piezo audio transducer at multiple frequencies ($frequency_1$, $frequency_2$, and $frequency_3$ in this example) and to calculate the power consumed by the piezo audio transducer at the different frequencies. In this example, the piezo audio transducer is driven at $frequency_1$ twice so that power consumption values at $frequency_1$ can be measured twice to reduce the probability of interference from other components, such as RF components. One of ordinary skill in the art will recognize that any one or more of the frequencies may be re-tested. The method 600 begins at block 610 by configuring and driving the piezo audio transducer at $frequency_1$ for a predetermined piezo sampling period (e.g., 100 ms, 150 ms, 175 ms, 200 ms, 500 ms, etc.) and measuring the corresponding voltage ($V_1$) and current ($I_1$), directly or indirectly (as discussed above). In block 620, the method calculates a first value for $P_1$ as the product of $V_1$ and $I_1$ minus $P_{back}$ (see blocks 415 and 460 above). In block 630, the method configures and drives the piezo audio transducer at $frequency_2$ and measures the corresponding voltage ($V_2$) and current ($I_2$). In block 640, the method calculates $P_2$ as the product of $V_2$ and $I_2$ minus $P_{back}$. As discussed above, power measurements at lower frequencies may be more susceptible to interference from other components, such as RF transmitters, etc. Thus, the piezo audio transducer is re-configured at the lowest frequency included in the test sequence ($frequency_1$ in this example). In block 650, the method re-configures the piezo audio transducer to be driven at $frequency_1$ and re-measures the voltage ($V_1$) and current ($I_1$). In block 660, the method calculates a second value for $P_1$ as the product of the re-measured values for $V_1$ and $I_1$ minus $P_{back}$. In block 670, the method determines P1 as the minimum value of the two power consumption values calculated for $frequency_1$ (i.e., the values calculated in blocks 620 and 660). In block 680, the method configures and drives the piezo audio transducer at $frequency_3$ and measures the corresponding voltage ($V_3$) and current ($I_3$). In block 690, the method calculates $P_3$ as the product of $V_3$ and $I_3$ minus $P_{back}$. The method then returns the calculated power values ($P_1$, $P_2$, and $P_3$) and processing continues at block 520.

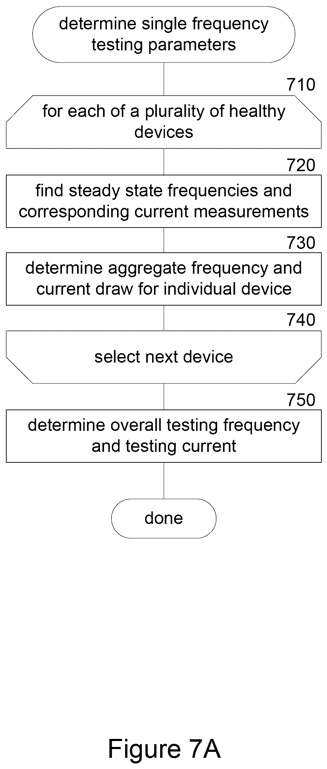

Figure 7A illustrates an example method 700 for establishing a test frequency and currency metric of a single frequency piezo audio transducer failure detection system, in accordance with aspects of the present disclosure. In this embodiment, piezo audio transducers are tested for failure using a single driving frequency. Typically, as the driving frequency increases, so does the amount of current drawn by the piezo audio transducer. However, at some point, the piezo audio transducer reaches a maximum current draw and increases in driving frequency have little to no impact on the amount of current drawn by the piezo audio transducer. In order to determine such a frequency for a given device (e.g., a particular piezo audio transducer product or line), a number of known healthy devices are evaluated by testing ever increasing driving frequencies until additional increase in frequency fail to significantly alter the amount of current drawn by the piezo audio transducer.

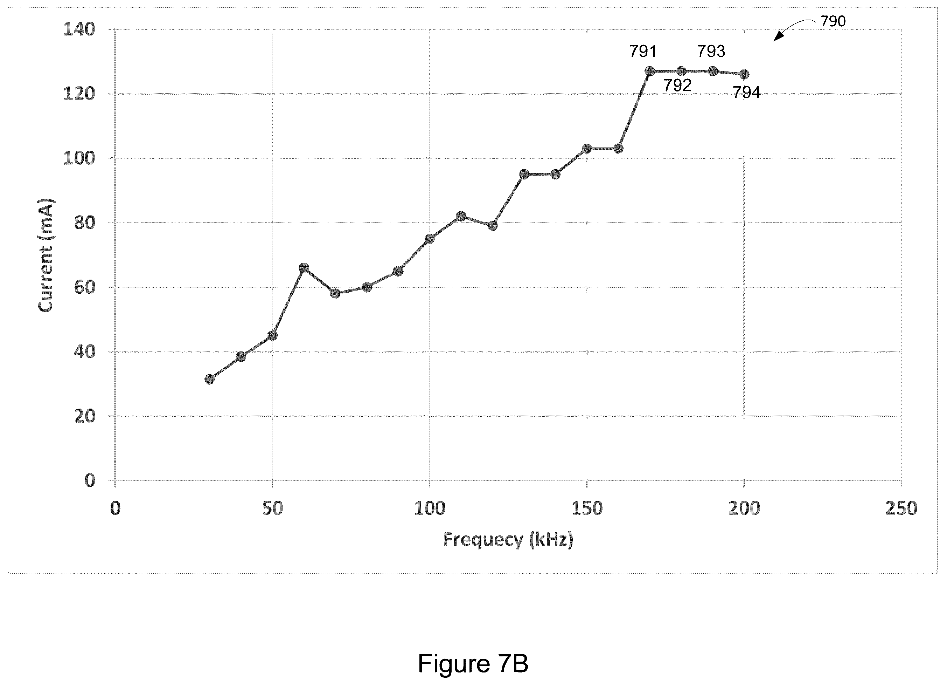

Figure 7B is a graph 790 illustrating the current draw (vertical axis) of an example piezo audio transducer at a number of driving frequencies (horizontal axis). In this example, the piezo audio transducer was driven at 10 kHz increments, starting at 30 kHz and the amount of current drawn at each driving frequency was measured (e.g., via a fuel gauging chip, PMIC, etc.). At 170 kHz, the current draw began to level out at approximately 127 mA and remained in a generally steady state (e.g., within 1 mA) for three more consecutive measurements.

Returning to Figure 7A, in blocks 710-740, each of a plurality of known (or presumed) healthy devices are assessed individually to identify driving frequencies that result in a steady state for the corresponding current draw. In block 720, a corresponding piezo audio transducer is driven at increasing frequencies until the current drawn by the piezo audio transducer levels out or reaches a steady state. For example, the method may determine that a steady state has been reached when a predetermined number (e.g., 3, 4, 10, 14) of consecutive measurements are within a predetermined threshold of each other (e.g., 0.1 mA, 0.5 mA, 1 mA, 5 mA, 1%, 10%). In block 730, after a steady state is reached, the corresponding driving frequencies and currency measurements are used to establish an aggregate frequency and aggregate current draw for the device based on the steady state frequencies and corresponding current measurements. For example, an aggregate frequency for the device could be determined based on a minimum frequency value, maximum frequency value, average frequency value (e.g., mean, media, mode), etc. Similarly, an aggregate current draw for the device could be determined based on a minimum current draw value, maximum current draw value, average current draw value (e.g., mean, media, mode), etc. Turning back to Figure 7B, four consecutive measurements 791-794 starting at 170 kHz were within 1 mA of each other. In this example, measurements 791-794 could be used to establish an aggregate frequency and aggregate current draw for the device in this example, such as 170 kHz (minimum frequency value) and 127 mA (mode of the current draw). As another example, the method may calculate the aggregate frequency as the maximum frequency (200 kHz) in the set of steady state frequencies and the aggregate current draw as the mean current draw (126.75 mA) for the set of steady state frequencies. One of ordinary skill in the art will recognize that aggregate values may be computed using any number of approaches to statistical analysis. In block 740, if there are any additional devices to be assessed the method loops back to block 710 to assess the next device. In block 750, the method determines an overall testing frequency and current draw by aggregating (e.g., minimum, maximum, average, etc.) the frequency and current draw values determined for each of the individual devices. In this manner, the testing frequency and current draw values are determined by assessing multiple devices to account for any variations that may exist in the individual devices.

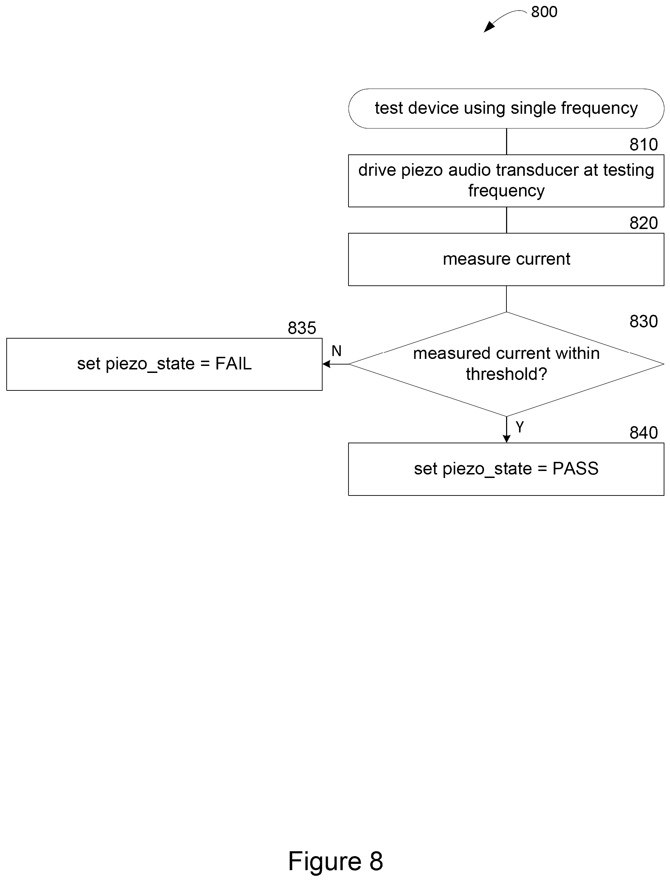

Figure 8 illustrates an example method 800 for testing a device using a single frequency, in accordance with aspects of the present disclosure. The method 800 begins at block 810, which involves driving the piezo audio transducer at the testing frequency, such as a testing frequency determined via method 700. In block 820, the current drawn by the piezo audio transducer while being driven at the testing frequency is measured. In decision block 830, the measured current draw is compared to a current draw threshold, such as a testing current draw determined via method 700 to determine a piezo health state or status for the piezo audio transducer. In block 835, if the measured current draw is within a predetermined threshold of the testing current draw (e.g., within 1 mA, 2 mA, 5 mA, 1%, 15%, etc.), then the method sets a piezo_state variable to PASS, otherwise in block 840 the method sets the piezo_state variable to FAIL. In some cases, the method may also include sending an alert to one or more parties or devices to indicate the audio transducer failed the test. For example, the method may send an alert or indication of the error to a paired device, such as a display screen, phone, tablet, etc. As another example, the method may send a message to a phone number or email address of the patient, the patient's caregiver, or another party. After the test is performed, the method may terminate or loop back to decision block 810 to perform the test periodically (e.g., every 3 hours, 8 hours, daily, weekly, monthly, etc.).

The embodiments disclosed herein are examples of the disclosure and may be embodied in various forms. For instance, while the embodiments have been described in view of an insulin infusion system, the invention can be used in any medical device having a piezo transducer. However, the embodiments disclosed herein may also be applied to non-medical device having a piezo transducer, such as smoke detectors, carbon monoxide detectors, mobile devices, etc. Further, although certain embodiments herein are described as separate embodiments, each of the embodiments herein may be combined with one or more of the other embodiments herein. Specific structural and functional details disclosed herein are not to be interpreted as limiting, but as a basis for the claims and as a representative basis for teaching one skilled in the art to variously employ the present disclosure in virtually any appropriately detailed structure. Like reference numerals may refer to like elements throughout the description of the figures.

Any of the herein described techniques, operations, methods, programs, algorithms, components, or codes may be converted to, or expressed in, a programming language or computer program embodied on a computer, processor, or machine-readable medium. The terms "programming language" and "computer program," as used herein, each include any language used to specify instructions to a computer or processor, and include (but is not limited to) the following languages and their derivatives: Assembler, Basic, Batch files, BCPL, C, C+, C++, Delphi, Fortran, Java, JavaScript, machine code, operating system command languages, Pascal, Perl, PL1, Python, scripting languages, Visual Basic, metalanguages which themselves specify programs, and all first, second, third, fourth, fifth, or further generation computer languages. Also included are database and other data schemas, and any other meta-languages. No distinction is made between languages which are interpreted, compiled, or use both compiled and interpreted approaches. No distinction is made between compiled and source versions of a program. Thus, reference to a program, where the programming language could exist in more than one state (such as source, compiled, object, or linked) is a reference to any and all such states. Reference to a program may encompass the actual instructions and/or the intent of those instructions. Reference to a component may encompass computer-executable instructions stored in one or more memories or other storage devices for execution by one or more processors or other processing devices.

It should be understood that the foregoing description is only illustrative of the present disclosure. To the extent consistent, any or all of the aspects detailed herein may be used in conjunction with any or all of the other aspects detailed herein. Various alternatives and modifications can be devised by those skilled in the art without departing from the disclosure. Accordingly, the present disclosure is intended to embrace all such alternatives, modifications, and variances. The embodiments described with reference to the attached drawing figures are presented only to demonstrate certain examples of the disclosure. Other elements, steps, methods, and techniques that are insubstantially different from those described above and/or in the appended claims are also intended to be within the scope of the disclosure.

The descriptions of embodiments of the technology are not intended to be exhaustive or to limit the technology to the precise form disclosed above. Where the context permits, singular or plural terms may also include the plural or singular term, respectively. Although specific embodiments of, and examples for, the technology are described above for illustrative purposes, various equivalent modifications are possible within the scope of the technology, as those skilled in the relevant art will recognize. For example, while steps are presented in a given order, alternative embodiments may perform steps in a different order. The various embodiments described herein may also be combined to provide further embodiments.

As used herein, the terms "generally," "substantially," "about," and similar terms are used as terms of approximation and not as terms of degree, and are intended to account for the inherent variations in measured or calculated values that would be recognized by those of ordinary skill in the art.

Moreover, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

While several embodiments of the disclosure have been depicted in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

Further disclosed herein is the subject-matter of the following clauses:

1. A medical device comprising:

a piezo audio transducer;
one or more processors; and
one or more processor-readable media storing instructions which, when executed by the one or more processors, cause the medical device to:

for each of a plurality of driving frequencies,

drive the piezo audio transducer at the driving frequency for a predetermined period, and
determine an amount of power consumed by the piezo audio transducer while being driven at the driving frequency for the predetermined period,

for a first pair of driving frequencies comprising a first driving frequency of the plurality of driving frequencies and a second driving frequency of the plurality of driving frequencies,
determine a slope for the first pair of driving frequencies based on the difference between the determined amount of power consumed by the piezo audio transducer while being driven at the first driving frequency and the determined amount of power consumed by the piezo audio transducer while being driven at the second driving frequency, and
for a second pair of driving frequencies comprising the first driving frequency and a third driving frequency of the plurality of driving frequencies,
determine a slope for the second pair of driving frequencies based on the difference between the determined amount of power consumed by the piezo audio transducer while being driven at the first driving frequency and the determined amount of power consumed by the piezo audio transducer while being driven at the third driving frequency, and
calculate a piezo health metric for the piezo audio transducer based on the calculated slopes.

2. The medical device of clause 1, the one or more processor-readable media further storing instructions which, when executed by the one or more processors, cause the medical device to measure an input current to a driver of the piezo audio transducer.

3. The medical device of clause 1 or 2, the one or more processor-readable media further storing instructions which, when executed by the one or more processors, cause the medical device to measure an amount of power consumed from a battery while the piezo audio transducer is operating.

4. The medical device of clause 1 or of one of clauses 1 to 3, wherein the determination of the slope for the first pair of driving frequencies is further based on the difference between the first driving frequency and the second driving frequency.

5. The medical device of clause 1 or of one of clauses 1 to 4, the one or more processor-readable media further storing instructions which, when executed by the one or more processors, cause the medical device to:

compare the calculated piezo health metric to a predetermined threshold;
determine whether the calculated piezo health metric is less than the predetermined threshold; and
after determining that the calculated piezo health metric is less than the predetermined threshold, alert a user.

6. The medical device of clause 1 or of one of clauses 1 to 5, the one or more processor-readable media further storing instructions which, when executed by the one or more processors, cause the medical device to:
display an indication of the calculated piezo health on a monitoring device.

7. The medical device of clause 1 or of one of clauses 1 to 6, the one or more processor-readable media further storing instructions which, when executed by the one or more processors, cause the medical device to:

disable one or more peripherals of the medical device; and
force one or more microcontroller units of the medical device into full active mode.

8. The medical device of any one of clause 1 or of one of clauses 1 to 7, wherein the medical device is a therapy delivery system.

9. The medical device of clause 1 or of one of clauses 1 to 8, wherein the medical device is an infusion system.

10. The medical device of clause 1 or of one of clauses 1 to 9, wherein the medical device is a medical monitoring system.

11. A computer-readable storage medium storing instructions that, when executed by a system having a piezo audio transducer for alerting a user, cause the system to perform operations for testing the piezo audio transducer, the operations comprising:

for each of a plurality of driving frequencies,

driving the piezo audio transducer at the driving frequency for a predetermined period, and
determining an amount of power consumed by the piezo audio transducer while being driven at the driving frequency for the predetermined period;

for a first pair of driving frequencies comprising a first driving frequency of the plurality of driving frequencies and a second driving frequency of the plurality of driving frequencies,
determining a slope for the first pair of driving frequencies based on the difference between the determined amount of power consumed by the piezo audio transducer while being driven at the first driving frequency and the determined amount of power consumed by the piezo audio transducer while being driven at the second driving frequency; and
for a second pair of driving frequencies comprising the first driving frequency of the plurality of driving frequencies and a third driving frequency of the plurality of driving frequencies,
determining a slope for the second pair of driving frequencies based on the difference between the determined amount of power consumed by the piezo audio transducer while being driven at the first driving frequency and the determined amount of power consumed by the piezo audio transducer while being driven at the third driving frequency; and
calculating a piezo health metric for the piezo audio transducer based on the determined slopes.

12. The computer-readable storage medium of clause 11, wherein determining the amount of power consumed by the piezo audio transducer comprises measuring an input current to a driver of the piezo audio transducer.

13. The computer-readable storage medium of clause 11 or 12, wherein determining the amount of power consumed by the piezo audio transducer comprises measuring an amount of power consumed from a battery while the piezo audio transducer is operating.

14. The computer-readable storage medium of clause 11 or of one of clauses 11 to 13, wherein determining the slope for the first pair of driving frequencies further based on the difference between the first driving frequency and the second driving frequency.

15. The computer-readable storage medium of clause 11 or of one of clauses 11 to 14, the operations further comprising:

comparing the calculated piezo health metric to a predetermined threshold; and
after determining that the calculated piezo health metric is less than the predetermined threshold, alerting a user.

16. The computer-readable storage medium of clause 15, wherein alerting the user comprises causing a message to be displayed on a monitoring device of the system.

17. The computer-readable storage medium of clause 11 or of one of clauses 11 to 16, wherein the system is an alert system, a smoke detector, a carbon monoxide detector, or a gas leak monitor.

18. The computer-readable storage medium of clause 11 or of one of clauses 11 to 17, wherein the system is a motion detector, an indoor air quality monitor, or a water leak detector.

19. A method for testing a piezo audio transducer, the method comprising:

driving the piezo audio transducer at a testing frequency;
measuring an amount of current drawn by the piezo audio transducer while being driven at the testing frequency;
comparing the measured amount of current drawn by the piezo audio transducer to a predetermined threshold; and
determining a piezo health status for the piezo audio transducer based on the comparison.

20. The method of clause 19, wherein the method is performed by a medical device having a processor, a memory, and a least one light emitting diode, the method further comprising:
alerting a user of the determined piezo health status at least in part by causing the light emitting diode to blink.

**Claims**

1. A method, performed in an insulin infusion system having one or more microcontroller units that include at least one processor and at least one memory, of testing a piezo audio transducer of the insulin infusion system, the method comprising:

disabling one or more peripherals of the insulin infusion system;
disconnecting one or more RF devices of the insulin infusion system;
forcing the one or more microcontroller units of the insulin infusion system into a full active mode;
for each of a plurality of driving frequencies of the full active mode,

driving the piezo audio transducer at the driving frequency for a predetermined period, and
determining an amount of power consumed by the piezo audio transducer while being driven at the driving frequency for the predetermined period;

for a first pair of driving frequencies comprising a first driving frequency of the plurality of driving frequencies and a second driving frequency of the plurality of driving frequencies,
determining a slope for the first pair of driving frequencies based on the difference between the determined amount of power consumed by the piezo audio transducer while being driven at the first driving frequency and the determined amount of power consumed by the piezo audio transducer while being driven at the second driving frequency; and
for a second pair of driving frequencies comprising the first driving frequency of the plurality of driving frequencies and a third driving frequency of the plurality of driving frequencies,
determining a slope for the second pair of driving frequencies based on the difference between the determined amount of power consumed by the piezo audio transducer while being driven at the first driving frequency and the determined amount of power consumed by the piezo audio transducer while being driven at the third driving frequency; and
calculating a piezo health metric for the piezo audio transducer based on the determined slopes.

2. The method of claim 1, wherein determining the amount of power consumed by the piezo audio transducer comprises measuring an input current to a driver of the piezo audio transducer.

3. The method of claim 1 or claim 2, wherein determining the amount of power consumed by the piezo audio transducer comprises measuring an amount of power consumed from a battery while the piezo audio transducer is operating.

4. The method of any one of claims 1 to 3, wherein determining the slope for the first pair of driving frequencies further based on the difference between the first driving frequency and the second driving frequency.

5. The method of any one of claims 1 to 4, further comprising:

comparing the calculated piezo health metric to a predetermined threshold; and
after determining that the calculated piezo health metric is less than the predetermined threshold, alerting a user.

6. The method of claim 5, wherein alerting the user comprises causing a message to be displayed on a monitoring device of the insulin infusion system.

7. A medical device comprising:

   a piezo audio transducer;
   one or more processors; and
   one or more processor-readable media storing instructions which, when executed by the one or more processors, cause the medical device to:

   for a first pair of driving frequencies comprising a first driving frequency of a plurality of driving frequencies and a second driving frequency of the plurality of driving frequencies,
   determine a slope for the first pair of driving frequencies based on the difference between the determined amount of power consumed by the piezo audio transducer while being driven at the first driving frequency and the determined amount of power consumed by the piezo audio transducer while being driven at the second driving frequency, and
   for a second pair of driving frequencies comprising the first driving frequency and a third driving frequency of the plurality of driving frequencies,
   determine a slope for the second pair of driving frequencies based on the difference between the determined amount of power consumed by the piezo audio transducer while being driven at the first driving frequency and the determined amount of power consumed by the piezo audio transducer while being driven at the third driving frequency, and
   calculate a piezo health metric for the piezo audio transducer based on the calculated slopes.

8. The medical device of claim 7, the one or more processor-readable media further storing instructions which, when executed by the one or more processors, cause the medical device to measure an input current to a driver of the piezo audio transducer.

9. The medical device of claim 7 or claim 8, the one or more processor-readable media further storing instructions which, when executed by the one or more processors, cause the medical device to measure an amount of power consumed from a battery while the piezo audio transducer is operating.

10. The medical device of any one of claims 7 to 9, wherein the determination of the slope for the first pair of driving frequencies is further based on the difference between the first driving frequency and the second driving frequency.

11. The medical device of any one of claims 7 to 10, the one or more processor-readable media further storing instructions which, when executed by the one or more processors, cause the medical device to:
    compare the calculated piezo health metric to a predetermined threshold.

12. The medical device of any one of claims 7 to 11, the one or more processor-readable media further storing instructions which, when executed by the one or more processors, cause the medical device to:
    display an indication of the calculated piezo health on a monitoring device.

13. The medical device of any one of claims 7 to 12, the one or more processor-readable media further storing instructions which, when executed by the one or more processors, cause the medical device to:

    disable one or more peripherals of the medical device;
    disconnect one or more RF devices of the medical device; and
    force one or more microcontroller units of the medical device into full active mode.

14. The medical device of any one of claims 7 to 13, wherein the medical device is a therapy delivery system.

15. The medical device of any one of claims 7 to 14, wherein the medical device is an infusion system.

FIG. 1

**FIG. 2**

Figure 3A

Figure 3B

start test

400

410 measuring voltage and current directly? — Y → 415 $P_{back} = 0$

N

420 disable non-essential system components and peripherals

430 disconnect non-essential RF devices

440 force all MCU cores into full active mode

450 measure background battery voltage ($V_{back}$) and current ($I_{back}$)

460 $P_{back} = V_{back} \times I_{back}$

470 calculate piezo health metric

480 piezo health metric > 1? — N → 485 set piezo_state = FAIL

Y

490 set piezo_state = PASS

Figure 4

calculate piezo health metric

500

510

determine power consumption values $(P_i)$ for different frequencies

520

$S_{12} = (P_2-P_1)/(frequency_2-frequency_1)$

530

$S_{13} = (P_3-P_1)/(frequency_3-frequency_1)$

540

$S_{23} = (P_3-P_2)/(frequency_3-frequency_2)$

550

calculate initial piezo health metric $(PHM_{INIT})$

560

$PHM_{INIT} \geq$ threshold

Y → return $PHM_{INIT}$

N

570

$S_{12} > 0$

Y → return $S_{12}^2 \times (S_{13} + S_{23})$

N

return $S_{13} + S_{23}$

Figure 5

Figure 6

determine single frequency
testing parameters

710

for each of a plurality of healthy
devices

720

find steady state frequencies and
corresponding current measurements

730

determine aggregate frequency and
current draw for individual device

740

select next device

750

determine overall testing frequency
and testing current

done

## Figure 7A

Figure 7B

EP 4 676 093 A1

800

test device using single frequency

810

drive piezo audio transducer at testing frequency

820

measure current

830

measured current within threshold?

835

set piezo_state = FAIL

N

Y

840

set piezo_state = PASS

Figure 8

# EUROPEAN SEARCH REPORT

**Application Number**

EP 25 18 5612

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2022/152294 A1 (BEGUIN STEVE [IE] ET AL) 19 May 2022 (2022-05-19)<br>* paragraphs [0007], [0043] - paragraph [0045]; figures 15-17 *<br>* paragraph [0050] - paragraph [0051] *<br>----- | 1-15 | INV.<br>H04R17/00<br>H04R29/00<br><br>ADD.<br>H04R1/02 |
| A | US 2015/086028 A1 (GAISER YVONNE [DE] ET AL) 26 March 2015 (2015-03-26)<br>* paragraphs [0006], [0022] - paragraph [0027] *<br>----- | 1-15 | |
| A | CN 113 630 707 A (HUAQIANG FANTAWILD SHENZHEN INTELLIGENT TECH CO LTD) 9 November 2021 (2021-11-09)<br>* abstract;<br>paragraph [0006] *<br>----- | 1-15 | |

**TECHNICAL FIELDS SEARCHED (IPC)**

H04R

The present search report has been drawn up for all claims

| Place of search | Date of completion of the search | Examiner |
|---|---|---|
| The Hague | 3 October 2025 | Will, Robert |

EPO FORM 1503 03.82 (P04C01)

## ANNEX TO THE EUROPEAN SEARCH REPORT
## ON EUROPEAN PATENT APPLICATION NO.

EP 25 18 5612

This annex lists the patent family members relating to the patent documents cited in the above-mentioned European search report.
The members are as contained in the European Patent Office EDP file on
The European Patent Office is in no way liable for these particulars which are merely given for the purpose of information.

03-10-2025

| Patent document cited in search report | Publication date | Patent family member(s) | | Publication date |
|---|---|---|---|---|
| US 2022152294 A1 | 19-05-2022 | AU | 2021381898 A1 | 22-06-2023 |
| | | CA | 3198761 A1 | 27-05-2022 |
| | | CN | 116457563 A | 18-07-2023 |
| | | EP | 4248557 A1 | 27-09-2023 |
| | | JP | 2023549553 A | 27-11-2023 |
| | | US | 2022152294 A1 | 19-05-2022 |
| | | WO | 2022108905 A1 | 27-05-2022 |
| US 2015086028 A1 | 26-03-2015 | CN | 104221400 A | 17-12-2014 |
| | | EP | 2837209 A1 | 18-02-2015 |
| | | JP | 6444298 B2 | 26-12-2018 |
| | | JP | 2015519787 A | 09-07-2015 |
| | | US | 2015086028 A1 | 26-03-2015 |
| | | WO | 2013153484 A1 | 17-10-2013 |
| CN 113630707 A | 09-11-2021 | NONE | | |

EPO FORM P0459

For more details about this annex : see Official Journal of the European Patent Office, No. 12/82

**REFERENCES CITED IN THE DESCRIPTION**

**Patent documents cited in the description**

- US 63666590